# EUROPÄISCHE PATENTSCHRIFT

(11) **EP 1 555 250 B1**
(45) Veröffentlichungstag und Bekanntmachung des Hinweises auf die Patenterteilung: **14.08.2013**
(21) Anmeldenummer: 05000185.8
(22) Anmeldetag: 07.01.2005
(51) Int. Cl.: C03C 25/32, C08G 18/28, C08G 18/70, C08G 18/12, C08G 18/08, C03C 25/26

(54) **Schlichtezusammensetzung**
Sizing composition
Composition d'encollage

(30) Priorität: 16.01.2004 DE 102004002527
(43) Veröffentlichungstag der Anmeldung: 20.07.2005
(73) Patentinhaber: Bayer Intellectual Property GmbH, 40789 Monheim (DE)
(72) Erfinder: Rische, Thorsten, Dr., 59423 Unna (DE); Feller, Thomas, 42659 Solingen (DE); Meixner, Jürgen, Dr., 47803 Krefeld (DE)
(74) Vertreter: BIP Patents

(56) Entgegenhaltungen:
- WO-A-03/060017
- US-A- 5 922 806

## Beschreibung

Die Erfindung betrifft neue thermovergilbungsstabile Schlichtezusammensetzungen, deren Herstellung und Verwendung.

Bei der Beschlichtung von Glas- und Kohlefasern werden wie z.B. in EP-A 792 900 u.a. PUR-Dispersionen als Bindemittelkomponenten in der Schlichtezusammensetzung verwendet.

Bedingt durch die vergleichsweise hohen Temperaturen bei den Beschichtungs- und Trocknungsprozessen sowie bei der Eincompoundierung der beschlichteten Glasfaser in eine Kunststoffmatrix, die z. T. deutlich mehr als 200°C betragen können, kommt es oftmals zu unerwünschter Thermovergilbung der hergestellten Beschichtungen.

Aus dem Stand der Technik sind zahlreiche Stabilisatoren und Additive bekannt, die eine Thermovergilbung von Bindemitteln verringern können. Diese Systeme haben im Bereich wässriger PUR-Dispersion allerdings eine nur unzureichende vergilbungshemmende Wirkung oder führen zu schlechteren anwendungstechnischen Eigenschaften der Dispersionen und Beschichtungen wie schlechteres Zug-Dehnungsverhalten oder schlechte Verträglichkeiten gegenüber anderen Lack- bzw. Schlichtekomponenten. Ferner neigen die bekannten Additive zu Migration aus den hergestellten Beschichtungen, so dass im Laufe der Zeit unerwünschtes Fogging und eine nachlassende Vergilbungsstabilisierung eintritt.

US-A 5,137,967 beschreibt die Herstellung thermovergilbungsstabiler, carboxylathaltiger PUR-Dispersionen, die nach dem sogenannten Prepolymer-Mischverfahren hergestellt werden. Zur Vergilbungsstabilisierung wird Hydrazin zur Kettenverlängerung des Prepolymers und Dimethylaminoethanol (DMAE) als Neutralisationsamin für die Carbonsäuregruppen verwendet.

In der DE 32 38 169 wird ein Verfahren zur Herstellung von PUR-Dispersionen beschrieben, bei dem Hydrazin oder Hydrazide als Additive oder als Kettenverlängerer eingesetzt wird. Es werden ausschließlich anionische, carboxylatfunktionelle PUR-Dispersionen nach dem Prepolymer-Mischverfahren beschrieben.

Grundsätzlich sind Hydrazine und Hydrazide als Kettenverlängerer in Polyurethanen beispielsweise aus US-A 4 147 679 oder DE-A 23 14 513 bekannt. Zum Teil werden sie auch in Mischungen mit anderen Kettenverlängerern wie Diaminen eingesetzt (US-A 3 415 768). Sie dienen zur Verbesserung von Flexibilität, Härte, Beständigkeit und Trocknung der Beschichtungen.

Die Aufgabe der vorliegenden Erfindung bestand daher in der Bereitstellung von thermovergilbungsstabilen bzw. thermovergilbungsarmen Schlichtezusammensetzungen im Vergleich zu Schlichten des Stands der Technik.

Es wurde nun gefunden, dass Schlichten basierend auf speziell hergestellten PUR-Dispersionen unter Verwendung von Hydrazin als Kettenverlängerungskomponente, die gewünschten Eigenschaften ohne den Zusatz von speziellen externen Stabilisatoren/Additiven erfüllen.

Gegenstand der vorliegenden Erfindung sind daher Schlichtezusammensetzungen enthaltend
I) ein oder mehrere Polyurethan-Polyharnstoffdispersionen (PUR-Dispersionen)
II) gegebenenfalls weitere filmbildende Harze
III) gegebenenfalls Vernetzer
IV) Hilfs- und Zusatzstoffe,
dadurch gekennzeichnet, dass die in I) eingesetzten PUR-Dispersionen erhältlich sind, indem
A) zunächst ein NCO-gruppenhaltiges Polyurethanprepolymer durch Umsetzung von
   A1) Polyisocyanaten mit
   A2) polymeren Polyolen und/oder Polyaminen mit zahlenmittleren Molekulargewichten von 400 bis 8 000 g/mol,
   A3) gegebenenfalls niedermolekularen Verbindungen mit zahlenmittleren Molekulargewichten von 17 - 400 g/mol ausgewählt aus der Gruppe bestehend aus Mono- und Polyalkoholen, Mono- und Polyaminen sowie Aminoalkoholen,
   A4) isocyanatreaktiven, ionisch oder potentiell ionisch hydrophilierenden Verbindungen und/oder
   A5) isocyanatreaktiven nichtionisch hydrophilierenden Verbindungen
   A6) gegebenenfalls in aliphatischen Ketonen als Lösemittel
      mit der Maßgabe hergestellt wird, dass in keiner der Komponenten A1) bis A5) primäre oder sekundäre Aminogruppen enthalten sind,
B) das aus Schritt A) erhaltene Prepolymer entweder in aliphatischen Ketonen gelöst oder, sofern die Herstellung bereits in Anwesenheit von A6) durchgeführt wurde, die Prepolymerlösung gegebenenfalls durch weitere Zugabe aliphatischer Ketone verdünnt wird und
C) die noch freien NCO-Gruppen des Prepolymers mit einer Kettenverlängerungskomponente enthaltend
   C1) Hydrazin und/oder Hydrazinhydrat und
   C2) gegebenenfalls Verbindungen entsprechend der Definition der Komponenten A2), A3), A4) und/oder A5)
      mit der Maßgabe umgesetzt werden, dass
      - die Verbindungen der Komponente C2) primäre und/oder sekundäre Aminogruppen aufweisen,
      - die Gesamtmengen von C1) und C2) so bemessen sind, dass ein rechnerischer Kettenverlängerungsgrad von 101 bis 150 % erreicht wird und
      - das Mengenverhältnis von C1) und C2) so bemessen ist, dass mindestens 40 % der freien Isocyanatgruppen mit Aminogruppen aus der Komponente C1) kettenverlängert bzw. terminiert werden.

Geeignete Polyisocyanate der Komponente A1) sind die dem Fachmann an sich bekannten aromatischen, araliphatischen, aliphatischen oder cycloaliphatischen Polyisocyanate, welche auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen können. Diese können in A1) einzeln oder in beliebigen Mischungen untereinander eingesetzt werden.

Beispiele für geeignete aromatische, araliphatische, aliphatische oder cycloaliphatische Polyisocyanate sind durch Phosgenierung oder nach phosgenfreien Verfahren, beispielsweise durch thermische Urethanspaltung, zugängliche Di- bzw. Triisocyanate des Molekulargewichtsbereichs 140 bis 400 g/mol mit aliphatisch, cycloaliphatisch, araliphatisch und/oder aromatisch gebundenen Isocyanatgruppen, wie 1,4-Diisocyanatobutan, 1,5-Diisocyanatopentan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocya-nat, IPDI), 4,4'-Diisocyanatodicyclohexylmethan (Desmodur^{®} W, Bayer AG, Leverkusen), 4-Isocyanatomethyl-1,8-octandiisocyanat (Triisocyanatononan, TIN), ω,ω'-Diisocyanato-1,3-dimethylcyclohexan (H₆XDI), 1-Isocyanato-1-methyl-3-isocyanato-methylcyclohexan, 1-Isocyanato-1-methyl-4-isocyanato-methylcyclohexan, Bis-(isocyanatomethyl)-norbornan, 1,5-Naphthalen-diisocyanat, 1,3- und 1,4-Bis-(2-isocyanato-prop-2-yl)-benzol (TMXDI), 2,4- und 2,6-Diisocyanatotoluol (TDI) insbesondere das 2,4 und das 2,6-Isomere und technische Gemische der beiden Isomeren, 2,4'- und 4,4'-Diisocyanatodiphenylmethan (MDI), 1,5-Diisocyanatonaphthalin, 1,3-Bis(isocyanato-methyl)benzol (XDI) sowie beliebige Mischungen genannter Verbindungen.

Bevorzugt werden in A1) Polyisocyanate oder Polyisocyanatgemische der vorstehend genannten Art mit ausschließlich aliphatisch und/oder cycloaliphatisch gebundenen Isocyanatgruppen eingesetzt.

Besonders bevorzugt sind Hexamethylendiisocyanat, Isophorondiisocyanat, und die isomeren Bis-(4,4'-isocyanatocyclohexyl)methane sowie deren Mischungen.

Wesentlich ist, dass zur Prepolymerherstellung nur solche Verbindungen in A2) - A5) eingesetzt werden, die keine primären und/oder sekundären Aminofunktionen aufweisen. Im Rahmen der Kettenverlängerung hingegen können in C2) Verbindungen eingesetzt werden, die den Definitionen der Komponenten A2) - A5) entsprechen, zusätzlich aber primäre und/oder sekundäre Aminogruppen aufweisen.

Polymere Polyole oder Polyamine entsprechend der Definition der Komponente A2) stammen typischerweise aus der Gruppe der Polyacrylate, Polyester, Polylactone, Polyether, Polycarbonate, Polyestercarbonate, Polyacetale, Polyolefine und Polysiloxane und verfügen bevorzugt über eine Funkionalität bezogen auf gegenüber NCO-Gruppen reaktiven Funktionalitäten von 1,5 bis 4.

Besonders bevorzugt sind polymere Polyole der vorstehend genannten Art mit einem zahlenmittleren Molekulargewicht von 600 bis 2 500 g/mol und mit einer OH-Funktionalität von 2 bis 3.

Hydroxylgruppen aufweisende Polycarbonate entsprechend der Definition der Komponente A2) sind durch Reaktion von Kohlensäurederivaten, z.B. Diphenylcarbonat, Dimethylcarbonat oder Phosgen mit Diolen erhältlich.

Als derartige Diole kommen z.B. Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A, Tetrabrombisphenol A aber auch Lacton-modifizierte Diole in Frage. Bevorzugt enthält die Diolkompomponente 40 bis 100 Gew.-% Hexandiol, bevorzugt 1,6-Hexandiol und/oder Hexandiol-Derivate, besonders bevorzugt solche Derivate, die neben endständigen OH-Gruppen Ether- oder Estergruppen aufweisen, wie Produkte, die durch Umsetzung von 1 Mol Hexandiol mit mindestens 1 Mol, bevorzugt 1 bis 2 Mol Caprolacton gemäß der DE-A 17 70 245 oder durch Veretherung von Hexandiol mit sich selbst zum Di- oder Trihexylenglykol erhalten wurden. Die Herstellung solcher Derivate ist z.B. aus der DE-A 15 70 540 bekannt. Auch die in der DE-A 37 17 060 beschriebenen Polyether-Polycarbonatdiole können eingesetzt werden.

Die Hydroxylpolycarbonate sind bevorzugt linear, können jedoch gegebenenfalls durch den Einbau polyfunktioneller Komponenten, insbesondere niedermolekularer Polyole, verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, und Sorbit, Methylglykosid, 1,3,4,6-Dianhydrohexite.

Als Polyetherpolyole entsprechend der Definition der Komponente A2) geeignet sind die in der Polyurethanchemie an sich bekannten Polytetramethylenglykolpolyether, die z.B. über Polymerisation von Tetrahydrofuran durch kationische Ringöffnung hergestellt werden können.

Darüber hinaus geeignete Polyetherpolyole sind Polyether, wie die unter Verwendung von Startermolekülen hergestellten Polyole aus Styroloxid, Propylenoxid, Butylenoxid oder Epichlorhydrin, insbesondere des Propylenoxids.

Als Polyesterpolyole entsprechend der Definition der Komponente A2) geeignet sind z.B. Umsetzungsprodukte von mehrwertigen, bevorzugt zweiwertigen und gegebenenfalls zusätzlich dreiwertigen Alkoholen mit mehrwertigen, bevorzugt zweiwertigen Carbonsäuren. Anstelle der freien Polycarbonsäuren können auch die entsprechenden Polycarbonsäureanhydride oder entsprechende Polycarbonsäureester von niedrigeren Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome substituiert und/oder ungesättigt sein.

Im erfindungsgemäßen Verfahren können Verbindungen entsprechend der Definition der Komponente A3) zur Terminierung des Polyurethan-Prepolymers zugesetzt werden.

Hierzu geeignete Verbindungen sind beispielsweise aliphatische Monoalkohole oder Monoamine des genannten Molekulargewichtsbereichs mit 1 bis 18 C-Atomen, wie z.B. Ethanol, n-Butanol, Ethylenglykol-monobutylether, 2-Ethylhexanol, 1-Octanol, 1-Dodecanol, 1-Hexadecanol, Diethylamin, Dibutylamin, Ethanolamin, N-Methylethanolamin, N,N-Diethanolamin, Amine der Jeffamin® M-Reihe (Huntsman Corp. Europe, Belgien) oder aminofunktionelle Polyethylenoxide und Polypropylenoxide.

Darüber hinaus können Polyole, Aminopolyole oder Polyamine mit einem zahlenmittleren Molekulargewicht unter 400 g/mol im erfindungsgemäßen Verfahren eingesetzt werden. Beispielhaft zu nennen sind:
a) Alkandiole bzw. -triole, wie Ethandiol, 1,2- und 1,3-Propandiol, 1,4- und 2,3-Butandiol, 1,5-Pentandiol, 1,3-Dimethylpropandiol, 1,6-Hexandiol, Neopentylglykol, 1,4-Cyclohexandimethanol, 2-Methyl-1,3-propandiol, 2-Ethyl-2-butylpropandiol, Trimethylpentandiol, stellungsisomere Diethyloctandiole, 1,2- und 1,4-Cyclohexandiol, hydriertes Bisphenol A [2,2-Bis(4-hydroxycyclohexyl)propan], 2,2-Dimethyl-3-hydroxypropionsäure-(2,2-dimethyl-3-hydroxypropylester), Trimethylolethan, Trimethylol-propan oder Glycerin,
b) Etherdiole, wie Diethylendiglykol, Triethylenglykol, Tetraethylenglykol, Dipropylenglykol, Tripropylenglykol, 1,3-Butylenglykol oder Hydrochinon-dihydroxyethylether,
c) Esterdiole der allgemeinen Formeln (I) und (II),

   HO-(CH₂)ₓ-CO-O-(CH₂)_{y}-OH (I),

   HO-(CH₂)ₓ-O-CO-R-CO-O(CH₂)ₓ-OH (II),

   in welchen
   - R: ein Alkylen- oder Arylenrest mit 1 bis 10 C-Atomen, bevorzugt 2 bis 6 C-Atomen,
   - x: 2 bis 6 und
   - y: 3 bis 5 ist,
   wie z.B. δ-Hydroxybutyl-ε-hydroxy-capronsäureester, ω-Hydroxyhexyl-γ- hydroxybuttersäureester, Adipinsäure-(β-hydroxyethyl)ester und Terephthal-säure-bis(β-hydroxy-ethyl)-ester und
d) Di- und Polyamine wie z.B. 1,2-Diaminoethan, 1,3 Diaminopropan, 1,6-Diaminohexan, 1,3- und 1,4-Phenylendiamin, 4,4'-Diphenylmethandiamin, Isophorondiamin, Isomerengemisch von 2,2,4- und 2,4,4-Trimethylhexa-methylendiamin, 2-Methyl-pentamethylendiamin, Diethylen-triamin, 1,3- und 1,4-Xylylendiamin, α,α,α',α'-Tetramethyl-1,3- und -1,4-xylylendiamin, 4,4-Diaminodicyclohexylmethan, aminofunktionelle Polyethylenoxide oder Polypropylenoxide, die unter dem Namen Jeffamin^{®}, D-Reihe (Fa. Huntsman Corp. Europe, Belgien) erhältlich sind, Diethylentriamin und Triethylentetramin. Als Diamine im Sinne der Erfindung sind auch substituierte Hydrazine geeignet, wie z.B. N-Methylhydrazin, N,N'-Dimethylhydrazin und deren Homologe sowie Säuredihydrazide der Adipinsäure, β-Methyladipinsäure, Sebacinsäure, Hydracrylsäure und Terephthalsäure, Semicarbazido-alkylenhydrazide, wie z.B. β-Semicarbazidopropionsäurehydrazid (z.B. beschrieben in der DE-A 17 70 591), Semicarbazidoalkylen-carbazinester, wie z.B. 2-Semicarbazidoethylcarbazinester (z.B. beschrieben in der DE-A 19 18 504) oder auch Aminosemicarbazid-Verbindungen, wie z.B. β-Aminoethylsemicarbazido-carbonat (z.B. beschrieben in der DE-A 19 02 931).

Unter ionisch bzw. potentiell ionisch hydrophilierenden Verbindungen werden sämtliche Verbindungen verstanden, die mindestens eine isocyanatreaktive Gruppe sowie mindestens eine Funktionalität, wie z.B. -COOY, -SO₃Y, -PO(OY)₂ (Y beispielsweise = H, NH₄⁺, Metallkation), -NR₂, -NR₃⁺ (R = H, Alkyl, Aryl), aufweisen, die bei Wechselwirkung mit wässrigen Medien ein ggf. pH-Wert-abhängiges Dissoziationsgleichgewicht eingeht und auf diese Weise negativ, positiv oder neutral geladen sein kann.

Bevorzugte isocyanatreaktive Gruppen sind Hydroxyl- oder Aminogruppen.

Geeignete ionisch oder potentiell ionisch hydrophilierende Verbindungen entsprechend der Definition der Komponente A4 sind z.B. Mono- und Dihydroxycarbonsäuren, Mono- und Diaminocarbonsäuren, Mono- und Dihydroxysulfonsäuren, Mono- und Diaminosulfonsäuren sowie Mono- und Dihydroxyphosphonsäuren oder Mono- und Diaminophosphonsäuren und ihre Salze wie Dimethylolpropionsäure, Dimethylolbuttersäure, Hydroxypivalinsäure, N-(2-Aminoethyl)-β-alanin, 2-(2-Amino-ethylamino)-ethansulfonsäure, Ethylendiamin-propyl- oder -butylsulfonsäure, 1,2- oder 1,3-Propylendiamin-β-ethylsulfonsäure, Äpfelsäure, Zitronensäure, Glykolsäure, Milchsäure, Glycin, Alanin, Taurin, Lysin, 3,5-Diaminobenzoesäure, ein Additionsprodukt von IPDI und Acrylsäure (EP-A 0 916 647, Beispiel 1) und dessen Alkali- und/oder Ammoniumsalze; das Addukt von Natriumbisulfit an Buten-2-diol-1,4, Polyethersulfonat, das propoxylierte Addukt aus 2-Butendiol und NaHSO₃, z.B. beschrieben in der DE-A 2 446 440 (Seite 5-9, Formel I-III) sowie Verbindungen, die in kationische Gruppen überführbare, z.B. aminbasierende, Bausteine wie N-Methyl-diethanolamin als hydrophile Aufbaukomponenten enthalten. Weiterhin kann Cyclohexylaminopropansulfonsäure (CAPS) wie z.B. in der WO 01/88006 als Verbindung entsprechend der Definition der Komponente A4) verwendet werden.

Bevorzugte ionische oder potentiell ionische Verbindungen sind solche, die über Carboxy- oder Carboxylat- und/oder Sulfonatgruppen und/oder Ammoniumgruppen verfügen.

Besonders bevorzugte ionische Verbindungen sind solche, die Carboxyl- und/oder Sulfonatgruppen als ionische oder potentiell ionische Gruppen enthalten, wie die Salze von N-(2-Amino-Aminoethyl)-β-alanin, der 2-(2-Amino-ethylamino-)ethansulfonsäure oder des Additionsproduktes von IPDI und Acrylsäure (EP-A 0 916 647, Beispiel 1) sowie der Dimethylolpropionsäure.

Geeignete nichtionisch hydrophilierende Verbindungen entsprechend der Definition der Komponente A5) sind z.B. Polyoxyalkylenether, die mindestens eine Hydroxy- oder Aminogruppe enthalten. Diese Polyether enthalten einen Anteil von 30 Gew.-% bis 100 Gew.-% an Bausteinen, die vom Ethylenoxid abgeleitet sind. In Frage kommen linear aufgebaute Polyether einer Funktionalität zwischen 1 und 3, aber auch Verbindungen der allgemeinen Formel (III), 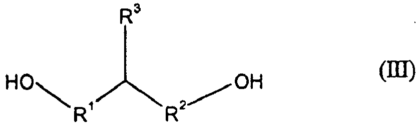 in welcher
- R¹ und R²: unabhängig voneinander jeweils einen zweiwertigen aliphatischen, cycloaliphatischen oder aromatischen Rest mit 1 bis 18 C-Atomen, die durch Sauerstoff und/oder Stickstoffatome unterbrochen sein können, bedeuten und
- R³: für einen alkoxyterminierten Polyethylenoxidrest steht.

Nichtionisch hydrophilierende Verbindungen sind beispielsweise auch einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylenoxideinheiten zu mindestens 30 mol-%, bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Im Verfahren wird bevorzugt eine Kombination aus ionischen und nicht-ionischen Hydrophilierungsmitteln entsprechend den Definitionen der Komponenten A4) und A5) verwendet. Besonders bevorzugt sind Kombinationen aus nichtionischen und anionischen Hydrophilierungsmitteln.

Zur Kettenverlängerung in Schritt C) werden Hydrazin und/oder dessen Hydrate als Komponente C1) eingesetzt. Bevorzugt ist die Verwendung von Hydrazin-Monohydrat.

Falls gewünscht können in Komponente C2) auch weitere Kettenverlängerer eingesetzt werden. Diese entsprechen den vorstehenden Definitionen der für A2) - A5) geeigneten Verbindungen mit der Maßgabe, dass die in C2) eingesetzten Verbindungen -NH₂ und/oder NH-Gruppen aufweisen.

Bevorzugt werden im Verfahren 7 bis 45 Gew.-% Komponente A1), 50 bis 91 Gew.-% Komponente A2), 0 bis 30 Gew.-% Verbindungen A3), 0 bis 12 Gew.-% Komponente A4), 0 bis 15 Gew.-% Komponente A5), 0,1 bis 5,0 Gew.-% C1) (bezogen auf reines Hydrazin N₂H₄) und 0 bis 15 Gew.-% C2) eingesetzt, wobei die Summe von A4) und A5) 0,1 bis 27 Gew.-% beträgt und sich die Summe aller Komponenten zu 100 Gew.-% addiert.

Besonders werden im Verfahren 10 bis 30 Gew.-% Komponente A1), 65 bis 90 Gew.-% Komponente A2), 0 bis 10 Gew.-% Komponente A3), 0 bis 10 Gew.-% Komponente A4), 0 bis 15 Gew.-% Komponente A5), 0,1 bis 3,0 Gew.-% C1) (bezogen auf reines Hydrazin N₂H₄) und 0 bis 10 Gew.-% C2) eingesetzt, wobei die Summe von A4) und A5) 0,1 bis 25 Gew.-% beträgt und sich die Summe aller Komponenten zu 100 Gew.-% addieren.

Ganz besonders bevorzugt werden im Verfahren 8 bis 27 Gew.-% Komponente A1), 65 bis 85 Gew.-% Komponente A2), 0 bis 8 Gew.-% Komponente A3), 0 bis 10 Gew.-% Komponente A4), 0 bis 15 Gew.-% Komponente A5), 1,0 bis 2,5 Gew.-% C1) (bezogen auf reines Hydrazin N₂H₄), und 0 bis 8 Gew.-% C2) eingesetzt, wobei die Summe von A4) und A5) 0,1 bis 25 Gew.-% beträgt und sich die Summe der Komponenten zu 100 Gew.-% addieren.

Das Verfahren zur Herstellung der wässrigen PUR-Dispersionen kann in einer oder mehreren Stufen in homogener, oder bei mehrstufiger Umsetzung, teilweise in disperser Phase durchgeführt werden. Nach vollständig oder teilweise durchgeführter Polyaddition aus A1) - A5) erfolgt ein Dispergier-, Emulgier- oder Lösungsschritt. Im Anschluss erfolgt gegebenenfalls eine weitere Polyaddition oder Modifikation in disperser Phase.

Zur Herstellung der wässrigen PUR-Dispersionen kann das aus dem Stand der Technik bekannte Aceton-Verfahren oder Abkömmlinge davon verwendet werden. Eine Zusammenfassung dieser Methoden findet sich in Methoden der organischen Chemie (Houben-Weyl, Erweiterungs- und Folgebände zur 4. Auflage, Band E20, H. Bartl und J. Falbe, Stuttgart, New York, Thieme 1987, S. 1671 - 1682). Bevorzugt ist das Aceton-Verfahren.

Üblicherweise werden im Verfahrensschritt A) die Bestandteile A2) bis A5), die keine primären oder sekundären Aminogruppen aufweisen dürfen und die Polyisocyanatkomponente A1) zur Herstellung eines Polyurethan-Prepolymers ganz oder teilweise vorgelegt und gegebenenfalls mit einem mit Wasser mischbaren aber gegenüber Isocyanatgruppen inerten Lösungsmittel A6) verdünnt und auf höhere Temperaturen, bevorzugt im Bereich von 50 bis 120°C, aufgeheizt.

Geeignete Lösungsmittel sind die üblichen aliphatischen ketofunktionellen Lösemittel wie z.B. Aceton, Butanon, die nicht nur zu Beginn der Herstellung, sondern gegebenenfalls in Teilen auch später zugegeben werden können. Bevorzugt sind Aceton und Butanon. Es ist möglich, die Reaktion unter Normaldruck oder erhöhtem Druck, z.B. oberhalb der Normaldruck-Siedetemperatur eines Lösungsmittels wie z.B. Aceton durchzuführen.

Weiterhin können im Verfahren die zur Beschleunigung der Isocyanatadditionsreaktion bekannten Katalysatoren, wie z.B. Triethylamin, 1,4-Diazabicyclo-[2,2,2]-octan, Dibutylzinnoxid, Zinndioktoat oder Dibutylzinndilaurat, Zinn-bis-(2-ethylhexanoat) oder andere metallorganischen Verbindungen mit vorgelegt oder später zudosiert werden. Bevorzugt ist Dibutylzinndilaurat.

Anschließend werden die gegebenenfalls zu Beginn der Reaktion noch nicht zugegebenen Bestandteile von A1) - A5) zudosiert.

Bei der Herstellung des Polyurethan-Prepolymeren in Schritt A) beträgt das Stoffmengenverhältnis von Isocyanatgruppen zu mit Isocyanat reaktiven Gruppen 1,0 bis 3,5, bevorzugt 1,1 bis 3,0, besonders bevorzugt 1,1 bis 2,5.

Die Umsetzung der Komponenten A1) - A5) zum Prepolymer erfolgt teilweise oder vollständig, bevorzugt aber vollständig. Der Umsetzungsgrad wird üblicherweise durch Verfolgung des NCO-Gehalts der Reaktionsmischung überwacht. Dazu können sowohl spektroskopische Messungen, z.B. Infrarot- oder Nahinfrarot-Spektren, Bestimmungen des Brechungsindex als auch chemische Analysen, wie Titrationen, von entnommenen Proben vorgenommen werden. Es werden so Polyurethan-Prepolymere, die freie Isocyanatgruppen enthalten, in Substanz oder in Lösung erhalten.

Nach oder während der Herstellung der Polyurethan-Prepolymere aus A1) und A2) bis A5) erfolgt, falls dies noch nicht in den Ausgangsmolekülen durchgeführt wurde, die teilweise oder vollständige Salzbildung der anionisch und/oder kationisch dispergierend wirkenden Gruppen. Im Falle anionischer Gruppen werden dazu Basen wie Ammoniak, Ammoniumcarbonat oder -hydrogencarbonat, Trimethylamin, Triethylamin, Tributylamin, Diisopropylethylamin, Dimethylethanolamin, Diethylethanolamin, Triethanolamin, Kaliumhydroxid oder Natriumcarbonat eingesetzt, bevorzugt Triethylamin, Triethanolamin, Dimethylethanolamin oder Diisopropylethylamin.

Die Stoffmenge der Basen liegt zwischen 50 und 100 %, bevorzugt zwischen 60 und 90 % der Stoffmenge der anionischen Gruppen. Im Falle kationischer Gruppen werden Schwefelsäuredimethylester oder Bernsteinsäure eingesetzt. Werden nur nichtionisch hydrophilierte Verbindungen A5) mit Ethergruppen verwendet, entfällt der Neutralisationsschritt. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt B), falls noch nicht oder nur teilweise unter A) geschehen das erhaltene Prepolymer mit Hilfe von aliphatischen Ketonen wie Aceton oder Butanon gelöst.

Im Verfahrensschritt C) werden die Komponente C1) sowie mögliche NH₂- und/oder NHfunktionelle Komponenten C2) mit den noch verbliebenen Isocyanatgruppen umgesetzt. Diese Kettenverlängerung/-terminierung kann dabei entweder in Lösungsmittel vor dem Dispergieren, während des Dispergierens oder in Wasser nach dem Dispergieren durchgeführt werden.

Werden zur Kettenverlängerung in C2) Verbindungen entsprechend der Definition von A4) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung in C1) und ggf. C2) eingesetzten Verbindungen zu freien NCO-Gruppen des Prepolymers liegt zwischen 101 - 150 %, wobei C1) in einer Menge zuzusetzen ist, dass mindestens 40 %, bevorzugt mindestens 50 % und besonders bevorzugt mindestens 70 % der NCO-Gruppen mit Verbindungen der Komponente C1) umgesetzt sind.

Auch für die Terminierung des Prepolymers können in C2) auch Monoamine wie z.B. Diethylamin, Dibutylamin, Ethanolamin, N-Methylethanolamin oder N,N-Diethanolamin verwendet werden.

Die aminischen Komponenten C1) und ggf. C2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mitverwendet werden so beträgt der Verdünnungsmittelgehalt bevorzugt 70 bis 95 Gew.-%.

Bevorzugt wird zur Kettenverlängerung erst die Komponente C1) mit den Verbindungen aus C2) entsprechend der Definition von A4) zugegeben und anschließend erst mit den Verbindungen aus C2) entsprechend den Definitionen von A2) und/oder A3) versetzt.

Üblicherweise erfolgt die Herstellung der PUR-Dispersionen aus den Prepolymeren im Anschluss an die Kettenverlängerung (Schritt C)). Dazu wird das gelöste und kettenverlängerte Polyurethanpolymer gegebenenfalls unter starker Scherung, wie z.B. starkem Rühren, entweder in das Dispergierwasser eingetragen oder man rührt umgekehrt das Dispergierwasser zu den Prepolymerlösungen. Bevorzugt wird das Wasser in das gelöste Prepolymer gegeben.

Grundsätzlich kann nach dem Dispergierschritt eine weitere Kettenverlängerung durch Zugabe weiterer Mengen C1) und C2) durchgeführt werden, bevorzugt wird die Kettenverlängerung aber ausschließlich vor der Dispergierung durchgeführt.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Die so erhaltenen Dispersionen haben einen Festkörpergehalt von 10 bis 70 Gew.-%, bevorzugt 25 bis 65 Gew.-% und besonders bevorzugt 30 bis 65 Gew.-%.

Je nach Neutralisationsgrad und Gehalt ionischer Gruppen kann die Dispersion sehr feinteilig eingestellt werden, so dass sie praktisch das Aussehen einer Lösung hat, aber auch sehr grobteilige Einstellungen sind möglich, die ebenfalls ausreichend stabil sind.

Weiterhin ist es möglich, die erhältlichen wässrigen PUR-Dispersionen durch Polyacrylate zu modifizieren. Hierzu wird in diesen Polyurethan-Dispersionen eine Emulsionspolymerisation von olefinisch ungesättigten Monomeren, z.B. Estern aus (Meth)acrylsäure und Alkoholen mit 1 bis 18 C-Atomen, Styrol, Vinylestern oder Butadien durchgeführt, wie es zum Beispiel in der DE-A 19 53 348, EP-A 0 167 188, EP-A 0 189 945 und EP-A 0 308 115 beschrieben ist.

Neben einer oder mehreren olefinische Doppelbindungen können diese Monomere auch funktionelle Gruppen wie Hydroxyl-, Epoxy-, Methylol- oder Acetoacetoxygruppen enthalten.

Als filmbildende Harze der Komponente II) sind die dem Fachmann an sich bekannten in Wasser dispergierbaren, emulgierbaren oder lösliche Polymere geeignet. Beispiele sind Polyesterpolymere oder Epoxidgruppen enthaltende Polyesterpolymere, Polyurethane, Acrylpolymere, Vinylpolymere wie Polyvinylacetat, Polyurethandispersionen, Polyacrylatdispersionen, Polyurethan-Polyacrylat-Hybriddispersionen, Polyvinylether- bzw. Polyvinylesterdispersionen, Polystyrol- bzw. Polyacrylnitrildispersionen.. Der Feststoffgehalt der filmbildenden Harze beträgt typischerweise 10 bis 95 Gew.-%, bevorzugt 30 bis 95 Gew.-%.

Die PUR-Dispersionen der Komponente I) wie auch die filmbildenden Harze der Komponente II) können gegenüber der Vernetzerkomponente III) reaktive Gruppen aufweisen.

Als Vernetzer der Komponente III) können Polyisocyanate mit gegebenenfalls blockierten NCO-Gruppen und/oder Aminovernetzerharze wie z.B. Melaminharze eingesetzt werden. Bevorzugt ist die Verwendung von hydrophilen bzw. hydrophilierten wasserlöslichen bzw. wasserdispergierbaren blockierten Polyisocyanaten in der Vernetzerkomponente III).

In einer bevorzugten Ausführungsform der Erfindung wird neben der Komponente I) mindestens ein Vernetzer und/oder filmbildendes Harz mitverwendet.

Als Komponente IV) werden den Schlichtezusammensetzungen Hilfs- und Zusatzstoffe zugegeben. Dies können Haftvermittler, Gleitmittel, Antistatika aber auch die dem Fachmann an sich gut bekannten Lackadditive wie Farbstoffe, Pigmente, Verlaufshilfsmittel, Licht- und Alterungsschutzmittel, UV-Absorber und so weiter sein. Eine Übersicht hierzu ist in K.L. Loewenstein "The Manufacturing Technology of Continous Glass Fibres", Elsevier Scientific Publishing Corp. Amsterdam, London, New York, 1983, Seite 243-295 gegeben.

Als Haftvermittler können in IV) die bekannten Silan-Haftvermittler wie 3-Aminopropyltrimethoxy- bzw. -triethoxysilan, N-(2-Aminoethyl)-3-aminopropyltrimethoxysilan, 3-Glycidylpropyltrimethoxysilan, Vinyltrimethoxysilan, Vinyltriethoxysilan oder 3-Methacryloxypropyltriethoxysilan. Die Konzentration der Silanhaftvermittler in den erfindungsgemäßen Schichtezusammensetzungen beträgt bevorzugt 0,05 bis 2 Gew.-%, besonders bevorzugt 0,15 bis 0,85 Gew.-%, bezogen auf die gesamte Schlichtezusammensetzung.

Ferner können die erfindungsgemäßen Schlichtezusammensetzungen ein oder mehrere nichtionische und/oder ionische Gleitmittel als Teil von Komponente IV) enthalten, wie Polyalkylenglykolether von Fettalkoholen oder Fettaminen, Polyalkylenglykolether und Glycerinester von Fettsäuren mit 12 bis 18 Kohlenstoffatomen, Polyalkylenglykole, höhere Fettsäureamide mit 12 bis 18 Kohlenstoffatomen von Polyalkylenglykolen und/oder Alkylenaminen, quartäre Stickstoffverbindungen, z.B. ethoxylierte Imidazoliniumsalze, Mineralöle und Wachse. Das oder die Gleitmittel werden bevorzugt in einer Gesamtkonzentration von 0,05 bis 1,5 Gew.-% bezogen auf die gesamte Schlichtezusammensetzung angewendet.

Die erfindungsgemäßen Schlichtezusammesetzungen können auch ein oder mehrere Antistatika als Teil von Komponente IV) enthalten. Beispielweise sind zu nennen Lithiumchlorid, Ammoniumchlorid, Cr-III-Salze, organische Titanverbindungen, Arylalkylsulfate oder Sulfonate, Arylpolyglykolethersulfonate oder quartäre Stickstoffverbindungen. Die Antistatika werden bevorzugt in Konzentrationen von 0,01 bis 0,8 Gew.-% angewendet.

Die Herstellung der Schlichtezusammensetzungen kann nach den an sich bekannten Methoden erfolgen. Bevorzugt wird in einem geeigneten Mischbehälter Wasser vorgelegt und unter Rühren das Bindemittel (Komponente I)), der Härter (Komponente III)) und anschließend das Gleitmittel und gegebenenfalls weitere Hilfsmittel aus Komponente IV) zugesetzt. Danach wird der pH-Wert auf 5 - 7 eingestellt und ein Hydrolysat eines Haftvermittlers aus Komponente IV) zugesetzt. Nach einer weiteren Rührzeit von 15 Minuten ist die Schlichtezusammensetzung gebrauchsfertig und kann gegebenenfalls nach pH-Wert Anpassung appliziert werden.

Die Schlichtenzusammensetzungen können über beliebige Methoden, beispielsweise mittels Sprüh- oder Walzapplikatoren auf ein geeignetes Substrat appliziert und ausgehärtet werden.

Geeignete Substrate sind beispielsweise ausgewählt aus der Gruppe Metall, Holz, Glas, Glasfasern, Kohlefasern, Stein, keramische Mineralien, Beton, harte und flexible Kunststoffe der verschiedensten Arten, gewebten und nicht gewebten Textilien, Leder, Papier, Hartfasern, Stroh und Bitumen die vor der Beschlichtung gegebenenfalls auch mit üblichen Grundierungen versehen werden können. Bevorzugte Substrate sind Glasfasern, Kohlefasern, Metalle, Textilien und Leder. Besonders bevorzugte Substrate sind Glasfasern.

Für die beschlichteten Glasfasern sind sowohl die für die Glasseidenfabrikation verwendeten, bekannten Glastypen wie E-, A-, C-, und S- Glas nach DIN 1259-1 als auch die anderen an sich bekannten Erzeugnisse der Glasfaserhersteller geeignet. Unter den genannten Glastypen für die Herstellung von Endlosglasfasem besitzen die E-Glasfasem aufgrund ihrer Alkalifreiheit, hohen Zugfestigkeit und hohen Elastizitätsmodul die größte Bedeutung für die Verstärkung von Kunststoffen.

Das Verfahren zur Herstellung, das Verfahren der Beschlichtung und die Nachbearbeitung der Glasfasern ist bekannt und beispielsweise in K.L. Loewenstein "The Manufacturing Technology of Continous Glass Fibres", Elsevier Scientific Publishing Corp., Amsterdam, London, New York, 1983, beschrieben.

Üblicherweise werden die Schlichten auf die mit hoher Geschwindigkeit aus Spinndüsen gezogenen Glasfilamente sofort nach deren Erstarren, d.h. noch vor dem Aufwickeln aufgetragen. Es ist aber auch möglich, die Fasern im Anschluss an den Spinnprozess in einem Tauchbad zu beschlichten. Die beschlichteten Glasfasern können entweder nass oder trocken beispielsweise zu Schnittglas verarbeitet werden. Die Trocknung des End- oder Zwischenproduktes findet bei Temperaturen zwischen 50 bis 200°C, bevorzugt 90 bis 150°C statt. Unter Trocknung ist dabei nicht allein die Entfernung von anderen flüchtigen Bestandteilen zu verstehen, sondern z.B. auch das Festwerden der Schlichtebestandteile. Erst nach beendeter Trocknung hat sich die Schlichte in die fertige Überzugsmasse verwandelt. Der Anteil der Schlichte beträgt, bezogen auf die beschlichteten Glasfasern, bevorzugt 0,1 bis 5,0 Gew.-% besonders bevorzugt 0,1 bis 3,0 Gew.-% und ganz besonders bevorzugt 0,3 bis 1,5 Gew.-%.

Als Matrixpolymere, in die die so hergestellten beschlichteten Glasfaser eingearbeitet werden können, können eine Vielzahl von Thermoplasten bzw. duroplastisch härtbaren Polymeren verwendet werden. Beispielsweise sind als thermoplastische Polymere geeignet: Polyolefine wie Polyethylen oder Polypropylen, Polyvinylchlorid, Polymerisate wie Styrol/Acrylnitril-Copolymere, ABS, Polymethacrylat oder Polyoxymethylen, aromatische und/oder aliphatische Polyamide wie Polyamid-6 oder Polyamid-6,6, Polykondensate wie Polycarbonat, Polyethylenterephthalat, flüssig-kristalline Polyarylester, Polyarylenoxid, Polysulfon, Polyarylensulfid, Polyarylsulfon, Polyethersulfon, Polyarylether oder Polyetherketon oder Polyaddukte wie Polyurethane. Als duroplastisch härtbare Polymere seien beipielsweise genannt: Epoxidharze, ungesättigte Polyesterharze, Phenolharze, Aminharze, Polyurethanharze, Polyisocyanurate, Epoxid/Isocyanurat-Kombinationsharze, Furanharze, Cyanuratharze und Bismaleinimidharze.

### Beispiele

Soweit nicht abweichend angegeben sind alle Prozentangaben als Gewichtsprozent zu verstehen.
Diaminosulfonat:
NH₂-CH₂CH₂-NH-CH₂CH₂-SO₃Na (45 %ig in Wasser)

Die Bestimmung der Festkörpergehalte erfolgte nach DIN-EN ISO 3251. NCO-Gehalte wurden, wenn nicht ausdrücklich anders erwähnt volumetrisch gemäß DIN-EN ISO 11909 bestimmt. Die Yellowness-Indices wurden mit Hilfe der CIELAB-Methode (DIN 5033) ermittelt.

### Vernetzerdispersion:

147,4 g eines biuretgruppen-haltigen Polyisocyanates auf Basis von 1,6-Diisocyanatohexan (HDI) mit einem NCO-Gehalt von 23,0% wurde bei 40°C vorgelegt. Innerhalb von 10 min wurden 121,0 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) unter Rühren zudosiert. Anschließend heizte man das Reaktionsgemisch auf 90°C und rührt solange bei dieser Temperatur bis der theoretische NCO-Wert erreicht war. Nach Abkühlen auf 65°C wurden innerhalb von 30 min 62,8 g Butanonoxim unter Rühren so zugetropft, dass die Temperatur des Gemisches von 80°C nicht überschritten wurde. Die Dispergierung erfolgte anschließend durch Zugabe von 726,0 g Wasser (T = 20°C) bei 60°C innerhalb von 30 min. Die Nachrührzeit bei 40°C betrugt 1 h.

Man erhielt eine lagerstabile wässrige Dispersion des blockierten Polyisocyanats mit einem Festkörpergehalt von 30,0 %.

### Beisṕiel 1: Vergleichsbeispiel

Baybond^{®} PU 401 (anionisch und nichtionisch hydrophilierte PUR-Dispersion mit einem Festkörpergehalt von 40 % und einer mittleren Teilchengröße von 100 - 300 nm, Bayer AG, Leverkusen, DE)

### Beispiel 2:

306,0 g Polyester PE 170 HN (Polyesterpolyol, OH-Zahl 66 mg KOH/g, zahlenmittleres Molekulargewicht 1700 g/mol, Bayer AG, Leverkusen, DE), 13,5 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid^{®} Z (Dibutylzinndilaurat, Bayer AG, Leverkusen, DE) wurden auf 65°C aufgeheizt. Anschließend wurde bei 65°C innerhalb von 5 min ein Gemisch aus 91,0 g Isophorondiisocyanat und 71,0 g Aceton zugegeben und solange unter Rückfluss gerührt bis der theoretische NCO-Wert erreicht wurde. Das fertige Prepolymer wurde in 353,2 g Aceton bei 50°C gelöst und anschließend eine Lösung aus 12,4 g Hydrazinhydrat und 40,5 g Wasser innerhalb von 10 min zudosiert. Nach Zugabe von 17,7 g Diaminosulfonat innerhalb von 5 min ließ man 15 min nachrühren und dispergierte durch Zugabe von 584,9 g Wasser innerhalb von 10 min. Es folgte die Entfernung des Lösemittels durch Destillation im Vakuum und man erhielt eine lagerstabile Dispersion mit einem Festkörpergehalt von 40,0 %.

### Beispiel 3:

1530,0 g Polyester PE 170 (Polyesterpolyol, OH-Zahl 66 mg KOH/g, zahlenmittleres Molekulargewicht 1700 g/mol, Bayer AG, Leverkusen, DE), 67,5 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid® Z (Dibutylzinndilaurat, Bayer AG, Leverkusen, DE) wurden auf 65°C aufgeheizt. Anschließend wurde bei 65°C innerhalb von 5 min ein Gemisch aus 537,1 g Desmodur^{®} W (Bis-(4,4'-isocyanatocyclohexyl)methan, Bayer AG, Leverkusen, DE) und 355,0 g Aceton zugegeben und solange unter Rückfluss gerührt bis der theoretische NCO-Wert erreicht wurde. Das fertige Prepolymer wurde mit 1766,0 g Aceton bei 50°C gelöst und anschließend eine Lösung aus 50,0 g Hydrazinhydrat, 51,0 g Isophorondiamin und 401,3 g Wasser innerhalb von 10 min zudosiert. Nach Zugabe von 63,3 g Diaminosulfonat innerhalb von 5 min ließ man 15 min nachrühren und dispergierte durch Zugabe von 2915,0 g Wasser innerhalb von 10 min. Es folgte die Entfernung des Lösemittels durch Destillation im Vakuum und man erhielt eine lagerstabile Dispersion mit einem Festkörpergehalt von 40,0 %.

### Beispiel 4:

1468,8 g Polyester PE 170 HN (Polyesterpolyol, OH-Zahl 66 mg KOH/g, zahlenmittleres Molekulargewicht 1700 g/mol, Bayer AG, Leverkusen, DE), 64,8 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid® Z (Dibutylzinndilaurat, Bayer AG, Leverkusen, DE) wurden auf 65°C aufgeheizt. Anschließend wurde bei 65°C innerhalb von 5 min ein Gemisch aus 436,9 g Isophorondiisocyanat und 340,8 g Aceton zugegeben und solange unter Rückfluss gerührt bis der theoretische NCO-Wert erreicht wurde. Das fertige Prepolymer wurde mit 1695,4 g Aceton bei 50°C gelöst und anschließend eine Lösung aus 55,2 g Hydrazinhydrat, 24,5 g Isophorondiamin und 319,0 g Wasser innerhalb von 10 min zudosiert. Nach Zugabe von 60,8 g Diaminosulfonat innerhalb von 5 min ließ man 15 min nachrühren und dispergierte durch Zugabe von 2714,1 g Wasser innerhalb von 10 min. Es folgte die Entfernung des Lösemittels durch Destillation im Vakuum und man erhielt eine lagerstabile Dispersion mit einem Festkörpergehalt von 40,0 %.

### Beispiel 5:

1453,5 g Polyester PE 170 HN (Polyesterpolyol, OH-Zahl 66 mg KOH/g, zahlenmittleres Molekulargewicht 1700 g/mol, Bayer AG, Leverkusen, DE), 64,1 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid® Z (Dibutylzinndilaurat, Bayer AG, Leverkusen, DE) wurden auf 65°C aufgeheizt. Anschließend wurde bei 65°C innerhalb von 5 min ein Gemisch aus 432,3 g Isophorondiisocyanat und 343,9 g Aceton zugegeben und solange unter Rückfluss gerührt bis der theoretische NCO-Wert erreicht wurde. Das fertige Prepolymer wurde mit 2298,5 g Aceton bei 50°C gelöst und anschließend eine Lösung aus 40,6 g Hydrazinhydrat, 48,5 g Isophorondiamin und 421,1 g Wasser innerhalb von 10 min zudosiert. Nach Zugabe von 60,1 g Diaminosulfonat innerhalb von 5 min ließ man 15 min nachrühren und dispergierte durch Zugabe von 2608,4 g Wasser innerhalb von 10 min. Es folgte die Entfernung des Lösemittels durch Destillation im Vakuum und man erhielt eine lagerstabile Dispersion mit einem Festkörpergehalt von 40,0 %.

### Beispiel 6:

1499,4 g Polyester PE.170 HN (Polyesterpolyol, OH-Zahl 66 mg KOH/g, zahlenmittleres Molekulargewicht 1700 g/mol, Bayer AG, Leverkusen, DE), 66,2 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid^{®} Z (Dibutylzinndilaurat, Bayer AG, Leverkusen, DE) wurden auf 65°C aufgeheizt. Anschließend wurde bei 65°C innerhalb von 5 min ein Gemisch aus 446,0 g Isophorondiisocyanat und 355,0 g Aceton zugegeben und solange unter Rückfluss gerührt bis, mit Hilfe von Nah-Infrarot-Spektroskopie (NIR) inline ermittelt, der theoretische NCO-Wert erreicht wurde. Das fertige Prepolymer wurde mit 1766,0 g Aceton bei 50°C gelöst und anschließend eine Lösung aus 49,0 g Hydrazinhydrat, 50,0 g Isophorondiamin und 443,0 g Wasser innerhalb von 10 min zudosiert. Nach Zugabe von 62,0 g Diaminosulfonat innerhalb von 5 min ließ man 15 min rühren und dispergierte durch Zugabe von 2686,1 g Wasser innerhalb von 90 min. Während des Dispergierschrittes erfolgte gleichzeitig die Entfernung des Lösemittels durch parallele Destillation im Vakuum und man erhielt eine lagerstabile Dispersion mit einem Festkörpergehalt von 40,0 %.

### Beispiel 7:

342,0 g PolyTHF 2000 (Polyether auf Basis von Tetrahydrofuran, OH-Zahl 56 mg KOH/g, zahlenmittleres Molekulargewicht 2000 g/mol), 16,7 g Polyether LB 25 (monofunktioneller Polyether auf Ethylenoxid-/Propylenoxidbasis zahlenmittleres Molekulargewicht 2250 g/mol, OH-Zahl 25 mg KOH/g, Bayer AG, Leverkusen, DE) und 0,1 g Desmorapid® Z (Dibutylzinndilaurat, Bayer AG, Leverkusen, DE) wurden auf 65°C aufgeheizt. Anschließend wurde bei 65°C innerhalb von 5 min ein Gemisch aus 86,5 g Isophorondiisocyanat und 67,5 g Aceton zugegeben und solange unter Rückfluss gerührt bis der theoretische NCO-Wert erreicht wurde. Das fertige Prepolymer wurde mit 335,5 g Aceton bei 50°C gelöst und anschließend eine Lösung aus 9,2 g Hydrazinhydrat, 9,4 g Isophorondiamin und 73,7 g Wasser innerhalb von 10 min zudosiert. Nach Zugabe von 15,0 g Diaminosulfonat innerhalb von 5 min ließ man 15 min nachrühren und dispergierte durch Zugabe von 615,4 g Wasser innerhalb von 10 min. Es folgte die Entfernung des Lösemittels durch Destillation im Vakuum und man erhielt eine lagerstabile Dispersion mit einem Festkörpergehalt von 40,0 %.

### Beispiel 8: Vergleichsbeispiel

Wässrige Polyurethan-Dispersion nach DE-A 32 38 169, Beispiel 2 über Prepolymermischverfahren hergestellt. Kettenverlängerung erfolgte ebenfalls mit Hydrazinhydrat.

### Beispiel 9: Vergleichsbeispiel

Wässrige Polyurethan-Dispersion hergestellt nach US-A 5,137,967, Beispiel 1, ebenfalls nach dem Prepolymermischverfahren und unter Kettenverlängerung mit Hydrazinhydrat.

### Anwendungsbeispiele

Tabelle 1 gibt die Schlichtezusamensetzungen im einzelnen wider. Die Herstellung der Zusammensetzungen wurde wie folgt durchgeführt: in einem Mischbehälter wurde die Hälfte der angegebenen Wassermenge vorgelegt und unter Rühren nacheinander die erfindungsgemäßen PUR-Dispersionen, filmbildende Harze, Vemetzerdispersion und Gleitmittel (Breox® 50-A 140, BP-Chemicals, GB) zugesetzt. Danach wurde der pH-Wert mit Essigsäure auf 5 - 7 eingestellt und ein nach Angaben des Herstellers hergestelltes Hydrolysat aus 3-Aminopropyl-triethoxysilan (A1100, UCC, New York, USA) als wässrige Haftvermittlerlösung zugesetzt. Nach einer weiteren Rührzeit von 15 Minuten war die Schlichte gebrauchsfertig.

Anschließend wurde gegebenenfalls nach Anpassung des pH-Wertes auf 5 - 7 die Schlichtezusammensetzungen auf Glasfasern appliziert. Die so beschlichteten Glasfasern wurden anschließend geschnitten und getrocknet.

| | **Schlichte 1** Vergleich | **Schlichte 2** | **Schlichte 3** | **Schlichte 4** | **Schlichte 5** |
|---|---|---|---|---|---|
| **Wasser** | 42,0 kg | 42,0 kg | 42,0 kg | 42,0 kg | 42,0 kg |
| **PUR-Dispersion** | 11,5 kg | 11,5 kg | 11,5 kg | 11,5 kg | 11,5 kg |

| | Beispiel 1 | Beispiel 2 | Beispiel 3 | Beispiel 4 | Beispiel 5 |
|---|---|---|---|---|---|
| **Vernetzerdispersion** | 3,5 kg | 3,5 kg | 3,5 kg | 3,5 kg | 3,5 kg |
| **Haftvermittler** | 0,6 kg | 0,6 kg | 0,6 kg | 0,6 kg | 0,6 kg |
| **Gleitmittel** | 0,4 kg | 0,4 kg | 0,4 kg | 0,4 kg | 0,4 kg |
| **Wasser** | 42,0 kg | 42,0 kg | 42,0 kg | 42,0 kg | 42,0 kg |
| **Gesamt** | 100,0 kg | 100,0 kg | 100,0 kg | 100,0 kg | 100,0 kg |
| **Yellowness Index [YI]*** | 61 | 53 | 55 | 53 | 51 |

| | | | | | |
|---|---|---|---|---|---|
| * bezieht sich auf die mit der jeweiligen Schlichte hergestellten, geschnittenen und getrockneten Glasfaser | | | | | |

| | **Schlichte 6** | **Schlichte 7** | **Schlichte 8**** Vergleich | **Schlichte 9**** Vergleich |
|---|---|---|---|---|
| **Wasser** | 42,0 kg | 42,0 kg | 42,0 kg | 42,0 kg |
| **PUR-Dispersion** | 11,5 kg | 11,5 kg | 11,5 kg | 11,5 kg |

| | Beispiel 6 | Beispiel 7 | Beispiel 8 | Beispiel 9 |
|---|---|---|---|---|
| **Vernetzerdispersion** | 3,5 kg | 3,5 kg | 3,5 kg | 3,5 kg |
| **Haftvermittler** | 0,6 kg | 0,6 kg | 0,6 kg | 0,6 kg |
| **Gleitmittel** | 0,4 kg | 0,4 kg | 0,4 kg | 0,4 kg |
| **Wasser** | 42,0 kg | 42,0 kg | 42,0 kg | 42,0 kg |
| **Gesamt** | 100,0 kg | 100,0 kg | 100,0 kg | 100,0 kg |
| **Yellowness Index [YI]*** | 50 | 56 | 63 | 67 |

| | | | | |
|---|---|---|---|---|
| * bezieht sich auf die mit der jeweiligen Schlichte hergestellteh, geschnittenen und getrockneten Glasfasern ** bei der Herstellung der Schlichte wurde der Haftvermittler A1100 direkt eingesetzt; auf die Herstellung eines Hydrolysats wurde verzichtet | | | | |

Die ermittelten Yellowness Indices als Maß für die Gelbfärbung der beschlichteten Glasfasern belegen, dass die mit den erfindungsgemäßen Schlichtezusammensetzungen beschlichteten Glasfasern signifikant niedrigere Thermovergilbung aufweisen als die mit den Schlichtezusammensetzungen des Stands der Technik hergestellten Glasfasern.

## Patentansprüche

1. Schlichtezusammensetzungen enthaltend
I) ein oder mehrere Polyurethan-Polyharnstoffdispersionen (PUR-Dispersionen)
II) gegebenenfalls weitere filmbildende Harze
III) gegebenenfalls Vernetzer
IV) Hilfs- und Zusatzstoffe,
**dadurch gekennzeichnet, dass** die in I) eingesetzten PUR-Dispersionen erhältlich sind in dem
A) zunächst ein NCO-gruppenhaltiges Polyurethanprepolymer durch Umsetzung von
A1) Polyisocyanaten mit
A2) polymeren Polyolen und/oder Polyaminen mit zahlenmittleren Molekulargewichten von 400 bis 8 000 g/mol,
A3) gegebenenfalls niedermolekularen Verbindungen mit zahlenmittleren Molekulargewichten von 17 - 400 g/mol ausgewählt aus der Gruppe bestehend aus Mono- und Polyalkoholen, Mono- und Polyaminen sowie Aminoalkoholen,
A4) isocyanatreaktiven, ionisch oder potentiell ionisch hydrophilierenden Verbindungen und/oder
A5) isocyanatreaktiven nichtionisch hydrophilierenden Verbindungen "
A6) gegebenenfalls in inertenLösemitteln mit der Maßgabe hergestellt wird, dass in keiner der Komponenten A1) bis A5) primäre oder sekundäre Aminogruppen enthalten sind,
B) das aus Schritt A) erhaltene Prepolymer entweder in aliphatischen Ketonen gelöst oder, sofern die Herstellung bereits in inerten Lösemitteln durchgeführt wurde, dessen Lösung gegebenenfalls durch weitere Zugabe aliphatischer Ketone verdünnt wird und
C) die noch freien NCO-Gruppen des Prepolymers mit einer Kettenverlängerungskomponente enthaltend
C1) Hydrazin und/oder Hydrazinhydrat und
C2) gegebenenfalls Verbindungen entsprechend der Definition der Komponenten A2), A3), A4) und/oder A5)
mit der Maßgabe umgesetzt werden, dass
• die Verbindungen der Komponente C2) primäre und/oder sekundäre Aminogruppen aufweisen,
• die Gesamtmengen von C1) und C2) so bemessen sind, dass ein rechnerischer Kettenverlängerungsgrad von 101 bis 150 % erreicht wird und
• das Mengenverhältnis von C1) und C2) so bemessen ist, dass mindestens 40 % der freien Isocyanatgruppen mit Aminogruppen aus der Komponente C1) kettenverlängert bzw. terminiert werden.

2. Schlichtezusammensetzungen gemäß Anspruch 1, **dadurch gekennzeichnet, dass** bei der Herstellung der PUR-Dispersionen in Schritt B) und gegebenenfalls Schritt A) Aceton oder Butanon als Lösemittel eingesetzt wird.

3. Schlichtezusammensetzungen gemäß Anspruch 1 oder 2, **dadurch gekennzeichnet, dass** bei der Herstellung der PUR-Dispersionen in den Schritten A) - C) 8 bis 27 Gew.-% Komponente A1), 65 bis 85 Gew.-% Komponente A2), 0 bis 8 Gew.-% Komponente A3), 0 bis 10 Gew.-% Komponente A4), 0 bis 15 Gew.-% Komponente A5), 1,0 bis 2,5 Gew.-% C1) (bezogen auf reines Hydrazin N₂H₄), und 0 bis 8 Gew.-% C2) eingesetzt, wobei die Summe von A4) und A5) 0,1 bis 25 Gew.-% beträgt und sich die Summe der Komponenten zu 100 Gew.-% aufaddiert.

4. Formkörper und/oder Beschichtungen hergestellt unter Verwendung von Schlichtezusammensetzungen gemäß eineim der Ansprüche 1 bis 3.

5. Substrate beschichtet mit Beschichtungen hergestellt unter Verwendung von SchJichtezusammensetzungen gemäß einem der Ansprüche 1 bis 3.

6. Glasfasern hergestellt unter Verwendung von Schlichtezusammensetzungen gemäß einem der Ansprüche 1 bis 3.

## Claims

1. Size compositions comprising
I) one or more polyurethane-polyurea dispersions (PU dispersions)
II) optionally further film-forming resins
III) optionally crosslinkers
IV) auxiliaries and additives,
**characterized in that** the PU dispersions used in I) are obtainable by
A) first preparing an NCO-containing polyurethane prepolymer by reacting
A1) polyisocyanates with
A2) polymeric polyols and/or polyamines having number-average molecular weights of 400 to 8000 g/mol,
A3) optionally low molecular weight compounds having number-average molecular weights of 17-400 g/mol selected from the group consisting of mono- and polyalcohols, mono- and polyamines and also amino alcohols,
A4) isocyanate-reactive, ionically or potentially ionically hydrophilicizing compounds and/or
A5) isocyanate-reactive nonionically hydrophilicizing compounds
A6) optionally in inert solvents
with the proviso that none of components A1) to A5) contains primary or secondary amino groups,
B) either dissolving the prepolymer obtained from step A) in aliphatic ketones or, if preparation has already been carried out in inert solvents, diluting the prepolymer solution optionally by further addition of aliphatic ketones, and
C) reacting the remaining free NCO groups of the prepolymer with a chain extender component comprising
C1) hydrazine and/or hydrazine hydrate and
C2) optionally compounds meeting the definition of components A2), A3), A4) and/or A5), with the proviso that
• the compounds of component C2) contain primary and/or secondary amino groups,
• the total amounts of C1) and C2) are such that an arithmetic degree of chain extension of 101 to 150% is attained and
• the proportion of C1) and C2) is such that at least 40% of the free isocyanate groups are terminated by and/or chain-extended with amino groups from component C1).

2. Size compositions according to Claim 1, **characterized in that** in preparing the PU dispersions in step B) and optionally step A) acetone or butanone is used as solvent.

3. Size compositions according to Claim 1 or 2, **characterized in that** in preparing the PU dispersions in steps A) - C) use is made of 8 to 27% by weight of component A1), 65 to 85% by weight of component A2), 0 to 8% by weight of component A3), 0 to 10% by weight of component A4), 0 to 15% by weight of component A5), 1.0 to 2.5% by weight of C1) (based on pure hydrazine, N₂H₄), and 0 to 8% by weight of C2), the sum of A4) and A5) being 0.1 to 25% by weight and the sum of the components adding to 100% by weight.

4. Mouldings and/or coatings produced using size compositions according to any one of Claims 1 to 3.

5. Substrates coated with coatings produced using size compositions according to any one of Claims 1 to 3.

6. Glass fibres produced using size compositions according to any one of Claims 1 to 3.

## Revendications

1. Compositions d'encollage, contenant :
I) une ou plusieurs dispersions de polyuréthane-polyurée (dispersions de PUR),
II) éventuellement d'autres résines filmogènes,
III) éventuellement des agents de réticulation,
IV) des adjuvants et des additifs,
**caractérisées en ce que** les dispersions de PUR utilisées en I) peuvent être obtenues par
A) tout d'abord la fabrication d'un prépolymère de polyuréthane contenant des groupes NCO par mise en réaction de
A1) des polyisocyanates avec
A2) des polyols et/ou polyamines polymères de poids moléculaires moyens en nombre de 400 à 8 000 g/mol,
A3) éventuellement des composés de faible poids moléculaire ayant des poids moléculaires moyens en nombre de 17 à 400 g/mol choisis dans le groupe constitué par les mono- et polyalcools, les mono- et polyamines et les aminoalcools,
A4) des composés réactifs avec les isocyanates, hydrophilisants ioniques ou potentiellement ioniques et/ou
A5) des composés réactifs avec les isocyanates, hydrophilisants non ioniques,
A6) éventuellement dans des solvants inertes,
à condition qu'aucun groupe amino primaire ou secondaire ne soit contenu dans les composants A1) à A5),
B) la dissolution du prépolymère obtenu à l'étape A) dans des cétones aliphatiques ou, si la fabrication a déjà été réalisée dans des solvants inertes, la dilution de sa solution éventuellement par ajout supplémentaire de cétones aliphatiques, et
C) la mise en réaction des groupes NCO encore libres du prépolymère avec un composant allongeur de chaîne contenant
C1) de l'hydrazine et/ou de l'hydrate d'hydrazine et
C2) éventuellement des composés correspondant à la définition des composants A2), A3), A4) et/ou A5),
à condition que
- les composés selon le composant C2) comprennent des groupes amino primaires et/ou secondaires,
- les quantités totales de C1) et C2) soient déterminées de manière à ce qu'un degré d'allongement de chaîne calculé de 101 à 150 % soit atteint, et
- la proportion de C1) et C2) soit déterminée de manière à ce qu'au moins 40 % des groupes isocyanate libres soient allongés ou terminés avec des groupes amino du composant C1).

2. Compositions d'encollage selon la revendication 1, **caractérisées en ce que** de l'acétone ou de la butanone est utilisée en tant que solvant lors de la fabrication des dispersions de PUR à l'étape B) et éventuellement à l'étape A).

3. Compositions d'encollage selon la revendication 1 ou 2, **caractérisées en ce que** lors de la fabrication des dispersions de PUR aux étapes A) à C), 8 à 27 % en poids du composant A1), 65 à 85 % en poids du composant A2), 0 à 8 % en poids du composant A3), 0 à 10 % en poids du composant A4), 0 à 15 % en poids du composant A5), 1,0 à 2,5 % en poids de C1) (par rapport à l'hydrazine pure N₂H₄) et 0 à 8 % en poids de C2) sont utilisés, la somme de A4) et A5) étant de 0,1 à 25 % en poids et la somme des composants étant de 100 % en poids.

4. Corps moulés et/ou revêtements fabriqués en utilisant des compositions d'encollage selon l'une quelconque des revendications 1 à 3.

5. Substrats revêtus avec des revêtements fabriqués en utilisant des compositions d'encollage selon l'une quelconque des revendications 1 à 3.

6. Fibres de verres fabriquées en utilisant des compositions d'encollage selon l'une quelconque des revendications 1 à 3.
